# EUROPEAN PATENT SPECIFICATION

(11) **EP 3 842 026 B1**
(45) Date of publication and mention of the grant of the patent: **23.07.2025**
(21) Application number: 20801887.9
(22) Date of filing: 07.07.2020
(51) Int. Cl.: A61J 15/00, A61M 25/01

(54) **MEDICAL DEVICE FOR INSERTING A PROBE**
MEDIZINPRODUKT ZUM EINSETZEN EINER SONDE
DISPOSITIF MÉDICAL POUR L'INSERTION D'UNE SONDE

(30) Priority: 07.05.2019 ES 201930727 U
(43) Date of publication of application: 30.06.2021
(73) Proprietor: García Andres, Manuel David, 08210 Barbera Del Valles Barcelona (ES)
(72) Inventor: García Andres, Manuel David, 08210 Barbera Del Valles Barcelona (ES)
(74) Representative: Gallego Jiménez, José Fernando
(86) International application number: PCT/ES2020/070436
(87) International publication number: WO 2020/225472

(56) References cited:
- EP-A1- 1 731 083
- WO-A2-2015/049560
- WO-A2-2015/049560
- CN-A- 106 236 592
- CN-U- 205 095 226
- ES-T3- 2 106 071
- JP-A- 2005 040 579
- JP-A- 2015 167 847
- JP-A- 2015 167 847
- KR-A- 20180 032 451
- KR-A- 20180 032 451
- US-A- 5 845 634
- US-A1- 2011 060 192
- US-A1- 2013 006 057
- US-B1- 7 762 261

## Description

### Object of the invention

The invention is a novel type of device for medical use, which is intended to facilitate the insertion of a tube into a patient under anaesthesia from the nose or the mouth to the oesophagus. To do so, the device of the invention comprises tubing and an inlet body joined to the tubing, such that both elements are communicated internally through a hole, and once the device is located orally in the patient, the device enables that through the internal walls thereof, a tube progresses indiscriminately from the oral or nasal cavity to the oesophagus, without causing twists in the tube and/or erosions along the path thereof.

The present invention relates to medical devices for use in the insertion of a tube orally or nasally.

### Background of the invention

Gastric tubes (also called nasogastric tubes, or NG tubes) are probably one of the most widely used types of medical tubes in hospitals, especially in surgical anaesthetic settings and intensive care units. These types of tubes are used, for example, in abdominal laparoscopic surgeries, in bowel resections, in patients who are in intensive care units (ICU) with parenteral nutrition, in stomach pumping in emergency surgeries, or in other normal operations.

Currently, gastric tubes are placed blindly by specialists, in other words, using only previously learnt manoeuvres and with variable success. According to an exhaustive study, it is only possible to achieve successful intubation on the first attempt in 40 % to 60 % of cases. For this reason, currently, the placement of a tube can cause complications, some serious, highlighting that in repeated placement attempts the rate of complications increases with each attempt, these drawbacks increasing when inserting a tube into a patient in the ICU, who is sedated or in the operating room under general anaesthesia.

Therefore, the solutions currently available in these cases where a tube must be introduced through the airway consist of the use of devices, for example, a video laryngoscope or fibre optic bronchoscope, which are means with complicated assembly and handling, apart from being expensive and affordable for few hospitals or, for example, the use of laryngoscopes, which are enormously traumatic devices assisted by Magill forceps. Other solutions that are currently used are the use of devices not approved for this purpose and which are not endorsed for it, this being the case, for example, of slotted tracheal tubing, urological tubes, metal flexo guides, Nelaton tubes or by freezing the gastric tube itself.

Thus, and in light of the above, the medical device for the insertion of a tube object of the present invention improves everything known until now in this sector since the device enables the intubation of a patient indiscriminately through the nose or mouth to the oesophagus without causing erosion in the path thereof, further reducing all kinds of complications for the patient, and for this reason it enables the execution of the tube placement manoeuvre to be much easier than what was known until now, both for the patient and the specialist. Furthermore, it should be highlighted that currently there is no known device specifically designed to facilitate the inlet of a tube through the mouth or nose, and enable the advance thereof to the oesophagus without needing to use auxiliary means, such as the one presented by the device of the invention. To do so, the device is made up of tubing which enables the inlet of a tube from the mouth to the oesophagus; and an inlet body connected through a through hole to the tubing, which enables the inlet of a tube from the nose to the oesophagus; and all this, without the tube coming into direct contact with the particularly sensitive mucous membranes of the upper airway and digestive tract.

WO 2015049560 A2 discloses a medical device for assisting enteral tube insertion from the nose to the oesophagus of a patient. The device includes a holding member for holding the device and an insert member for insertion of the enteral tube into the oesophagus. US20130006057 A1 discloses an insertion aid device for use in oral suctioning and cleaning, nasal and oral tracheal suctioning, that can also be used in insertion of nasal and oral gastric tubes, feeding tubes, and other medical devices. Such device comprises a tongue depressor for providing improved viewing of the oral cavity and maintaining the patient's mouth in an open position.

See further, e.g., EP11731083 A1 and US2011060192 A1.

Taking into account all the aforementioned aspects, it is considered that the present invention introduces a technical solution which solves the problems described previously, achieving a type of device that is structurally simple, cheap to manufacture and easy to use, in a manner that is different and improved compared to what is known until now in the field of medicine.

The invention is defined in independent claim 1 and subsequent dependent claims 2 - 8.

The following is a detailed description of the invention which completes these general ideas introduced in this point.

### Description of the invention

The medical device for the insertion of a tube object of the present invention comprises tubing and an inlet body joined to the tubing at a point along the vertical dimension thereof, both elements further being in internal connection through a through hole, such that a tube can be introduced into the inside of the assembly both through an upper end of the tubing and through the inlet body.

The medical device is inserted orally into a patient who is under the effects of general anaesthesia, between the posterior pharynx and the upper third of the oesophagus, such that due to the configuration of the device the insertion of a tube through the nasal cavity or through the oral cavity of the patient is facilitated indiscriminately.

This device, which is preferably made of silicone or polyurethane, adapts to the body of the patient, being between the posterior pharynx and the upper third of the oesophagus and performing the function of a ramp so that a tube easily slides along the internal walls of the device, preventing direct contact of this tube with the mucous membranes of the airway and upper digestive tract, until it is located in the oesophagus without causing harm to the patient.

It should be specified that the device is designed so that any type of gastric tube (NG tube), gastro-duodenal tube, oesophageal Doppler tubes, oesophageal thermometers, duodenal tubes can be introduced through the walls thereof, in addition to being able to be used in order to facilitate the introduction of any other device which needs to be led from the oral or nasal cavity to the digestive tract in an easy and simplified manner.

For a better understanding of what is explained, a nasogastric tube is a tube which is introduced through a cavity of the nose, while an orogastric tube is a tube which is introduced through the mouth.

As previously mentioned, the device of the invention comprises tubing and an inlet body, such that the inlet body is joined to the tubing at a point along the vertical dimension thereof; the tubing and the inlet body also being in connection through a through hole which communicates them internally.

For this reason, the device has two accesses to the inside of the tubing, since, on the one hand, a tube can be introduced from the upper end of the tubing and come out through the lower end thereof; and on the other hand, a tube can be introduced through the inlet body and come out through the lower end of the tubing.

The inlet body is shaped like a truncated cone, one of the ends of the cone defining an inlet mouth for a tube coming from the nasal cavity capable of passing through the through hole. However, according to one example, not forming part of the present invention, the inlet body is shaped like a truncated ovoid, one of the ends of the body defining an inlet mouth for a tube coming from the nasal cavity capable of passing through the through hole.

In this manner, the inlet body has at the upper end thereof an inlet mouth which covers a larger surface than the lower portion. In this manner, the surface covered by the inlet mouth enables the tube to be introduced into the inlet body and subsequently guided to the oesophagus. The ovoid and conical shapes of the inlet body enable it to adapt to the anatomy of the patient, specifically the retropharynx.

Once again, preferably, the lower end of the inlet body defines an outlet mouth for a tube from either the nasal or oral cavity, the tubing extending from this outlet mouth in order to guide the tube.

According to one embodiment, the outlet mouth comprises an oblique cut on a distal end. In this manner, the introduction of the device into the patient is facilitated, since said oblique cut enables the progressive advance from the outlet mouth through the oesophagus.

Once the device is located between the posterior pharynx and the upper third of the oesophagus, a tube can be introduced indiscriminately through the mouth of the patient through the tubing of the device, which protrudes from the mouth of the patient; or it can be introduced through the nose, through the inlet body. Once the tube goes beyond the through hole, it comes out through the outlet mouth of the lower end of the inlet body.

In this manner, in both cases the device enables the introduction of a tube from the oral or nasal cavity of a patient to the digestive tract thereof, making the tube advance through the oesophagus without twisting and preventing erosion from being caused in the path thereof; it being notable that after this insertion the device can be removed from the inside the patient without needing to remove the tube.

Once the device object of the invention is located between the posterior pharynx and the upper third of the oesophagus of the patient, it does not completely block the pharynx, thus enabling the insertion of a tube into the trachea through the mouth or nose.

Preferably, the tubing of the device is a preformed tubing, with a curvature which facilitates the insertion of the device into the patient and maintains it inside the patient in a predetermined manner.

According to one embodiment, the tubing of the device has at least one bending point located in the vertical dimension thereof above the inlet body, which provides said tubing with a slight bend when it is introduced into the body of the patient.

Preferably, the tubing and the inlet body have a slotted channel along the entire vertical axis thereof which facilitates the withdrawal of the tube from the inside of the device, once this tube has been properly placed in the oesophagus. Furthermore, this slotted channel enables the device to be bent for the insertion and/or extraction thereof inside the patient.

Preferably, the inlet body is flexible so it can be introduced into the patient and resistant so that it does not collapse due to the pressure of the anterior and posterior walls of the oropharynx.

According to one embodiment, the inlet body comprises, in a ventral portion, a ramp-like projection, arranged such that it joins the inlet body with the tubing. In this manner, said projection guides the tube from the inside of said inlet body to the inside of said tubing through the through hole.

### Brief description of the figures

To better understand what has been set forth above, several drawings schematically depicting a practical embodiment only by way of non-limiting example are attached.
Figure 1 shows a perspective view of the medical device for the insertion of a tube object of the invention.
Figure 2 shows a cross-sectional view of the medical device in the use position in a patient, when the tube is inserted through the mouth.
Figure 3 shows a cross-sectional view of the medical device in the use position in a patient, when the tube is inserted through the nose.

### Description of a preferred embodiment

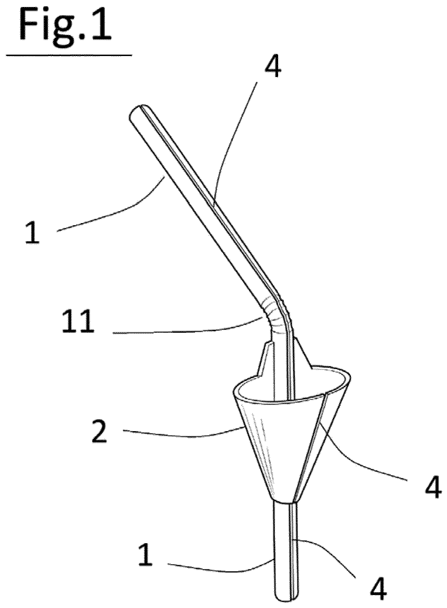

As seen in figures 1, 2 and 3, of a preferred embodiment of the invention, the medical device enables the introduction of a tube 5 from an oral or nasal cavity to the oesophagus 62 of a patient 6, and comprises tubing 1 and an inlet body 2, wherein the inlet body 2 has a conical shape and is joined to the tubing 1 at a point in the lower half of the referred-to vertical dimension of the tubing 1; both elements further being in connection through a through hole 3 which communicates them internally.

Specifically, figure 1 shows that along the entire vertical axis of the device, both in the tubing 1 and the inlet body 2, there is a slotted channel 4 through which the withdrawal of the assembly from the body of the patient is facilitated. Another noticeable detail in figure 1 is that the tubing 1 has a bending point 11 located in the vertical dimension thereof above the inlet body 2, which enables the tubing 1 to be bent or flexed, thus facilitating the location thereof when it is introduced by a specialist orally into a patient.

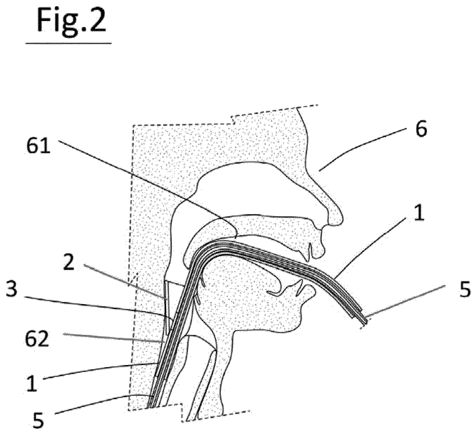

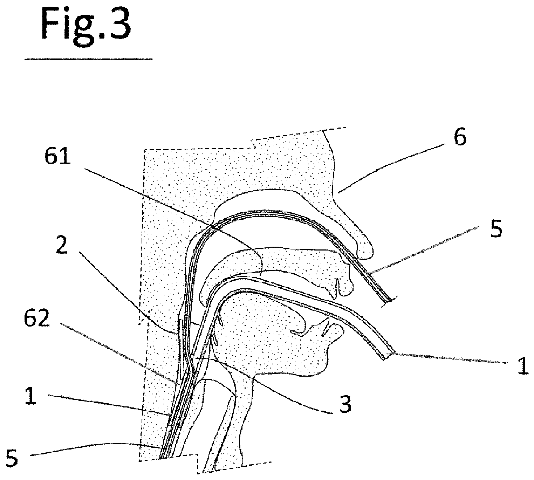

Moreover, both figure 2 and figure 3 show the device located in the body of a patient 6 between the posterior pharynx 61 and the upper third of the oesophagus 62. For this reason, both figures show how the device occupies the anatomical vacuum in the body of the patient, being capable of adapting to the anatomy of said region.

Thus, figure 2 shows in detail the device and the position occupied by a tube which has been introduced into a patient through the mouth to the oesophagus 62 through the device. In this case, as shown in this figure, the tube 5 is inserted into the device along the entire tubular cross section of the tubing 1, since the introduction of this tube 5 has been through the upper end of the tubing 1 or end of the tubing 1 protruding from the oral cavity of the patient 6. Therefore, in this figure it can be seen how by means of the device, the patient 6 is protected internally, from the posterior pharynx 61 to the oesophagus 62, from the possible damage that a tube 5 can produce when it is introduced from the oral cavity.

Moreover, figure 3 shows the representation of the device located in the body of a patient 6, and wherein it shows the position of a tube 5 through the device of the invention, which is introduced from the nose of the patient 6. In this representation, it is observed that the tube 5 is inserted into the device through the inlet body 2 of the device, which is communicated with the tubing 1 by means of a common through hole 3, such that when the tube 5 is introduced through the nasal cavities of the patient 6, this tube 5 is inserted into the device through the inlet body 2 to the end of tubing 1, which is housed in the oesophagus of the patient 62 or the lower end of the tubing 1. For this reason, in this figure it is seen how by means of the device, the patient 6 is protected internally, from the posterior pharynx 61 to the oesophagus 62, from the possible damage produced by a tube 5 when it is introduced from the nasal cavity.

Next, the operation of device 1 is described.

The device is introduced into the patient 6 through the oral cavity, sliding it over the surface of the hard palate, and once it has gone beyond the inlet body 2, it is deployed in the oropharynx, specifically between the posterior pharynx 61 and the upper third of the oesophagus 62. Once the device is in this position, the tube 5 can be introduced through the upper end of the tubing 1 or through the nasal cavity.

When introducing the tube 5 through the upper end of the tubing 1, the specialist moves it through the inside of the tubing until it reaches the lower end, where it comes out and is housed in the oesophagus 62. Before introducing it into the device, the tube 5 can be lubricated to facilitate the insertion thereof and movement through the tubing 1.

When the tube 5 is introduced through the nasal cavity, it runs through said cavity until it reaches the posterior portion of the pharynx where it is received by the inlet body 2. Once inside, the conical shape of the inlet body 2 guides the tube 5 to the through hole 3 in order to introduce it into the tubing 1. Once the tube 5 is introduced into the tubing 1, it continues the movement thereof until it reaches the lower end, through which it comes out and is housed in the oesophagus 62.

The device can be removed from inside the patient 6 without the tube 5 positioned in the oesophagus 62 needing to be removed. To do so, the device elastically deforms so that the slotted channel 4 enables the tube 5 to pass through it.

## Claims

1. A medical device for the insertion of a tube, with which a tube (5) is adapted to be introduced from the oral or nasal cavity to the oesophagus (62) of a patient (6); wherein the device comprises tubing (1) and an inlet body (2), **characterised in that** the inlet body (2) is shaped like a truncated cone with an upper end of the inlet body wider than a lower end of the inlet body, the inlet body further configured to be arranged between the posterior pharynx and the upper third of the oesophagus of a patient, and wherein said inlet body (2) is joined to the tubing (1) at a point along the vertical dimension thereof; the tubing (1) and the inlet body (2) also being in connection through a through hole (3) which communicates them internally, and wherein the inlet body has, at the upper end thereof, an inlet mouth configured to receive and guide a tube adapted to come from either the oral or nasal cavity and subsequently guided to the oesophagus.

2. The medical device for the insertion of a tube, according to claim 1, **characterised in that** the tubing (1) and the inlet body (2) have a slotted channel (4) along the vertical axis thereof.

3. The medical device for the insertion of a tube, according to claim 1, **characterised in that** the tubing (1) is preformed tubing.

4. The medical device for the insertion of a tube, according to claim 1, **characterised in that** the tubing (1) has at least one bending point (11) located in the vertical dimension thereof above the inlet body (2).

5. The medical device for the insertion of a tube, according to claim 1, wherein the lower end of the inlet body (2) defines an outlet mouth for a tube (5) coming from either the nasal or oral cavity, the tubing (1) extending from this outlet mouth in order to guide the tube (5).

6. The medical device for the insertion of a tube, according to claim 1, **characterised in that** the outlet mouth for a tube (5) comprises an oblique cut on a distal end.

7. The medical device for the insertion of a tube, according to claim 1, **characterised in that** the tubing (1) and the inlet body (2) are made of silicone.

8. The medical device for the insertion of a tube, according to claim 1, **characterised in that** the tubing (1) and the inlet body (2) are made of polyurethane.

## Patentansprüche

1. Medizinprodukt zum Einführen eines Schlauches, mit dem ein Schlauch (5) dazu angepasst ist, von der Mund- oder Nasenhöhle in die Speiseröhre (62) eines Patienten (6) eingeführt zu werden; wobei das Produkt ein Schlauchstück (1) und einen Einlasskörper (2) umfasst, **dadurch gekennzeichnet, dass** der Einlasskörper (2) wie ein Kegelstumpf geformt ist, wobei ein oberes Ende des Einlasskörpers breiter ist als ein unteres Ende des Einlasskörpers, wobei der Einlasskörper ferner dazu konfiguriert ist, zwischen dem hinteren Rachenraum und dem oberen Drittel der Speiseröhre eines Patienten angeordnet zu sein, und wobei der Einlasskörper (2) an einem Punkt entlang seiner vertikalen Ausdehnung mit dem Schlauchstück (1) verbunden ist; wobei das Schlauchstück (1) und der Einlasskörper (2) auch über ein Durchgangsloch (3) miteinander verbunden sind, das sie intern miteinander verbindet, und wobei der Einlasskörper an seinem oberen Ende eine Einlassöffnung aufweist, die dazu konfiguriert ist, einen Schlauch aufzunehmen und zu führen, der entweder aus der Mund- oder der Nasenhöhle kommt und anschließend zur Speiseröhre geführt wird.

2. Medizinprodukt zum Einführen eines Schlauches nach Anspruch 1, **dadurch gekennzeichnet, dass** das Schlauchstück (1) und der Einlasskörper (2) entlang ihrer vertikalen Achse einen geschlitzten Kanal (4) aufweisen.

3. Medizinprodukt zum Einführen eines Schlauches nach Anspruch 1, **dadurch gekennzeichnet, dass** das Schlauchstück (1) ein vorgeformtes Schlauchstück ist.

4. Medizinprodukt zum Einführen eines Schlauches nach Anspruch 1, **dadurch gekennzeichnet, dass** das Schlauchstück (1) mindestens einen Biegungspunkt (11) aufweist, der in seiner vertikalen Ausdehnung oberhalb des Einlasskörpers (2) angeordnet ist.

5. Medizinprodukt zum Einführen eines Schlauches nach Anspruch 1, wobei das untere Ende des Einlasskörpers (2) eine Auslassöffnung für einen Schlauch (5) definiert, der entweder aus der Nasen- oder der Mundhöhle kommt, wobei sich das Schlauchstück (1) von dieser Auslassöffnung aus erstreckt, um den Schlauch (5) zu führen.

6. Medizinprodukt zum Einführen eines Schlauches nach Anspruch 1, **dadurch gekennzeichnet, dass** die Auslassöffnung für einen Schlauch (5) einen schrägen Schnitt an einem distalen Ende umfasst.

7. Medizinprodukt zum Einführen eines Schlauches nach Anspruch 1, **dadurch gekennzeichnet, dass** das Schlauchstück (1) und der Einlasskörper (2) aus Silikon hergestellt sind.

8. Medizinprodukt zum Einführen eines Schlauches nach Anspruch 1, **dadurch gekennzeichnet, dass** das Schlauchstück (1) und der Einlasskörper (2) aus Polyurethan hergestellt sind.

## Revendications

1. Dispositif médical pour l'insertion d'un tube, avec lequel un tube (5) est adapté pour être introduit depuis la cavité buccale ou nasale vers l'œsophage (62) d'un patient (6) ; dans lequel le dispositif comprend une tubulure (1) et un corps d'entrée (2), **caractérisé en ce que** le corps d'entrée (2) a la forme d'un cône tronqué avec une extrémité supérieure du corps d'entrée plus large qu'une extrémité inférieure du corps d'entrée, le corps d'entrée est en outre configuré pour être agencé entre le pharynx postérieur et le tiers supérieur de l'œsophage d'un patient, et dans lequel ledit corps d'entrée (2) est joint à la tubulure (1) en un point le long de la dimension verticale de celle-ci ; la tubulure (1) et le corps d'entrée (2) étant également en liaison à travers un trou traversant (3) qui les fait communiquer intérieurement, et dans lequel le corps d'entrée présente, à l'extrémité supérieure de celui-ci, une bouche d'entrée configurée pour recevoir et guider un tube adapté pour provenir de la cavité buccale ou nasale et être ensuite guidé vers l'œsophage.

2. Dispositif médical pour l'insertion d'un tube, selon la revendication 1, **caractérisé en ce que** la tubulure (1) et le corps d'entrée (2) présentent un canal fendu (4) le long de l'axe vertical de ceux-ci.

3. Dispositif médical pour l'insertion d'un tube, selon la revendication 1, **caractérisé en ce que** la tubulure (1) est une tubulure préformée.

4. Dispositif médical pour l'insertion d'un tube, selon la revendication 1, **caractérisé en ce que** la tubulure (1) présente au moins un point de flexion (11) situé dans la dimension verticale de celle-ci au-dessus du corps d'entrée (2).

5. Dispositif médical pour l'insertion d'un tube, selon la revendication 1, dans lequel l'extrémité inférieure du corps d'entrée (2) définit une bouche de sortie pour un tube (5) provenant de la cavité nasale ou buccale, la tubulure (1) s'étendant depuis cette bouche de sortie afin de guider le tube (5).

6. Dispositif médical pour l'insertion d'un tube, selon la revendication 1, **caractérisé en ce que** la bouche de sortie pour un tube (5) comprend une découpe oblique sur une extrémité distale.

7. Dispositif médical pour l'insertion d'un tube, selon la revendication 1, **caractérisé en ce que** la tubulure (1) et le corps d'entrée (2) sont constitués de silicone.

8. Dispositif médical pour l'insertion d'un tube, selon la revendication 1, **caractérisé en ce que** la tubulure (1) et le corps d'entrée (2) sont constitués de polyuréthane.
